# EUROPÄISCHE PATENTSCHRIFT

(11) **EP 1 009 367 B1**
(45) Veröffentlichungstag und Bekanntmachung des Hinweises auf die Patenterteilung: **17.04.2002**
(21) Anmeldenummer: 98947476.2
(22) Anmeldetag: 29.08.1998
(51) Int. Cl.: A61K 7/06, A61K 7/00

(54) **VERWENDUNG EINER WIRKSTOFFKOMBINATION UND MITTEL ZUM SCHUTZ VON KERATINISCHEN FASERN**
USE OF A COMBINATION OF ACTIVE SUBSTANCES AND AGENT FOR PROTECTING KERATINIC FIBERS
UTILISATION D'UNE COMBINAISON DE PRINCIPES ACTIFS ET AGENT DE PROTECTION DES MATIERES KERATINIQUES

(30) Priorität: 02.09.1997 DE 19738303
(43) Veröffentlichungstag der Anmeldung: 21.06.2000
(73) Patentinhaber: Hans Schwarzkopf GmbH & Co. KG, 22763 Hamburg (DE)
(72) Erfinder: POPPE, Elisabeth, D-22299 Hamburg (DE); DELOWSKY, Jens, D-22844 Norderstedt (DE); EMMERLING, Winfried, D-25436 Tornesch (DE); BAUMSCHEIPER, Michael, D-22523 Hamburg (DE)
(74) Vertreter: Foitzik, Joachim Kurt
(86) Internationale Anmeldenummer: EP9805488
(87) Internationale Veröffentlichungsnummer: WO9911224

(56) Entgegenhaltungen:
- EP-A- 0 287 876
- EP-A- 0 786 250
- WO-A-87/03196
- WO-A-92/13829
- WO-A-96/10387
- WO-A-97/25973
- DE-A- 4 324 962
- FR-A- 2 700 954
- US-A- 4 777 037
- US-A- 5 756 106
- H. EGGENSPERGER ET AL : "Multiaktive Wirkstoffe für Kesmetika" SEIFEN, OLE, FETTE, WACHSE., Bd. 120, Nr. 16, 1994, Seiten 1013-1015, XP000483892 AUGSBURG DE

## Beschreibung

Die Erfindung betrifft die Verwendung einer speziellen Wirkstoffkombination zum Schutz von keratinischen Fasern vor Schäden durch Hitzeeinwirkung sowie diese Wirkstoffkombination enthaltende Mittel.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Wellmitteln. Tönungsmitteln und Stylingpräparaten. Während früher bei der Formulierung dieser Mittel vor allem diese Produkteigenschaften im Zentrum standen, wird nunmehr in immer größerem Maße angestrebt, mit diesen Mitteln auch eine Regeneration geschädigter Haare oder sogar einen präventiven Schutz vor Schädigungen zu erzielen.

Ein spezielles Problemfeld besteht dabei im Schutz insbesondere von empfindlichen oder anderweitig vorgeschädigten Haaren vor Schäden durch Hitzeeinwirkung. Solchen Hitzeeinwirkungen können Haare beispielsweise beim häufigen, intensiven Fönen, der Benutzung von Trockenhauben oder bei entsprechender Nutzung von Lockenstäben ausgesetzt sein.

Um den Zeit- und Arbeitsaufwand für den Anwender nicht durch Formulierung spezieller, zusätzlich anzuwendender Mittel zu erhöhen, konzentriert man die Entwicklung auf möglichst neue Wirkstoffe bzw. Wirkstoffkombinationen, die die gewünschte Wirkung im Rahmen der ohnehin angewendeten Mittel entfalten.

CA-2158538 offenbart die Verwendung einer Wirkstoffkombination, die Panthenol, Benzalkoniumchlorid und Cyclomethicon enthält, zum Schutz vor Schäden durch Hitzeeinwirkung.

Es wurde nun überraschenderweise gefunden, daß die Verwendung einer speziellen Wirkstoffkombination in üblichen Haarbehandlungsmitteln zu einem hervorragenden Schutz der Haare vor Schäden durch Hitzeeinwirkung führt.

Ein erster Gegenstand der Erfindung ist daher die Verwendung einer Wirkstoffkombination, bestehend aus mindestens einem faserstrukturverbessernden Wirkstoff A und mindestens einem konditionierenden Wirkstoff B ausgewählt aus der Gruppe, die aus Esterquats und Alkylamidoaminen gebildet wird, zum Schutz von keratinischen Fasern vor Schäden durch Hitzeeinwirkung.

Unter keratinischen Fasern werden erfindungsgemäß Pelze, Wolle. Federn und insbesondere menschliche Haare verstanden.

Eine erste zwingende Komponente der erfindungsgemäßen Wirkstoffkombination ist ein faserstrukturverbessernder Wirkstoff A.

Faserstrukturverbessernde Wirkstoffe sind dem Fachmann bekannt. Beispiele für solche Wirkstoffe sind
- tierische und pflanzliche Proteinhydrolysate, insbesondere Keratin- und pflanzliche Proteinhydrolysate,
- Vitamin und Vitaminvorstufen, wie Panthenol, dessen Derivate und Biotin,
- Mono-. Di- und Oligosaccharide,
- Honigextrakte,
- Ceramide

Bezüglich weiterer Verbindungen wird auf die dem Fachmann bekannten Handbücher, z. B. K. Schrader, Grundlagen und Rezepturen der Kosmetika, 2. Auflage, Hüthig Buch Verlag, Heidelberg, 1989, verwiesen.

Überraschenderweise wurde weiterhin gefunden, daß auch durch Anwendung vieler Pflanzenextrakte, insbesondere in Kombination mit weiteren faserstrukturverbessernden Wirkstoffen, eine, ggf. deutlich gesteigerte, faserstrukturverbessernde Wirkung erzielt werden kann. Pflanzenextrakte sind daher im Sinne der Erfindung ebenfalls zu den faserstrukturverbessernden Wirkstoffen zu zählen.

Eine erfindungsgemäß besonders geeignete Gruppe von faserstrukturverbessernden Wirkstoffen A1 stellen Panthenol und seine physiologisch verträglichen Derivate dar. Solche Derivate sind insbesondere die Ester und Ether des Panthenols sowie kationisch derivatisierte Panthenole. Einzelne Vertreter sind beispielsweise das Panthenoltriacetat. der Panthenolmonoethylether und dessen Monoacetat sowie die in der WO 92/13829 offenbarten kationischen Panthenolderivate. Panthenol ist innerhalb dieser Gruppe bevorzugt.

Eine weitere besonders geeignete Gruppe von faserstrukturverbessernden Wirkstoffen A2 sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflegeund Waschmittel e.V. (IKW), Frankfurt, beginnenden Tabelle aufgeführt sind.

Erfindungsgemäß sind vor allem die Extrakte aus Eichenrinde, Brennessel, Hamamelis, Hopfen. Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Apfel, Grapefruit, Salbei, Rosmarin. Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Eichenrinde, Brennessel, Hamamelis. Hopfen. Kamille, Klettenwurzel, Schachtelhalm, Lindenblüten, Mandel, Aloe Vera, Kokosnuß, Mango, Aprikose, Limone, Weizen, Kiwi, Melone, Orange, Apfel, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Mandel, Aloe Vera, Kokosnuß, Mango, Aprikose, Limone, Weizen, Kiwi und Melone.

Gemäß einer weiteren Ausführungsform der Erfindung können als Pflanzenextrakte auch die Extrakte von Wasserpflanzen, insbesondere von Algen, verwendet werden.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Ebenfalls erfindungsgemäß als faserstrukturverbessernde Wirkstoffe A bevorzugt sind Honigextrakte. Diese Extrakte werden in analoger Weise zu den Pflanzenextrakten gewonnen und enthalten üblicherweise 1 - 10 Gew.-%, insbesondere 3 - 5 Gew.-%, Aktivsubstanz. Wasser/Propylenglykol-Mischungen können auch hier bevorzugte Extraktionsmittel sein.

Ebenfalls faserstrukturverbessernde Wirkstoffe sind Proteinhydrolysate, insbesondere Elastin-. Kollagen-, Keratin-, Milcheiweiß-, Sojaprotein-, Mandelprotein- und Weizenproteinhydrolysate, deren Kondensationsprodukte mit Fettsäuren sowie quaternisierte Proteinhydrolysate.

Ebenfalls faserstrukturverbessernde Wirkstoffe sind Mono-, Di- und Oligosaccharide wie beispielsweise Glucose, Galactose, Fructose, Fruchtzucker und Lactose.

Eine zweite zwingende Komponente der erfindungsgemäßen Wirkstoffkombination ist ein konditionierender Wirkstoff B, ausgewählt aus der Gruppe, die aus Esterquats und Alkylamidoaminen gebildet wird.

Dem Fachmann sind die Verbindungen mit konditionierender Wirkung bekannt. Üblicherweise eingesetzte Verbindungen sind kationische Tenside, kationische Polymere, Alkylamidoamine, Paraffinöle, pflanzliche Öle und synthetische Öle wie Silikonöle. Bezüglich weiterer Verbindungen wird auf die dem Fachmann bekannten Handbücher, z.B. die oben genannte Monographie von K. Schrader, verwiesen.

Eine erfindungsgemäß besonders bevorzugte Gruppe von konditionierenden Wirkstoffen B1 stellen die sogenannten Esterquats dar.

Bei diesen Esterquats handelt es sich um bekannte Stoffe, die man nach den einschlägigen Methoden der präparativen organischen Chemie erhalten kann. In diesem Zusammenhang sei auf die Internationale Patentanmeldung WO 91/01295 verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Stellvertretend für den umfangreichen Stand der Technik sei an dieser Stelle auf die Druckschriften EP-A2 0 239 910, EP-A2 0 293 955 A2, EP-A2 0 295 739 und EP-A2 0 309 052 sowie die Übersichten von R.Puchta et al. in *Tens.Surf.Det.*, 30, 186 (1993), M.Brock in *Tens. Surf. Det.*, 30, 394 (1993) und R.Lagerman et al. in *J.Am.Oil.Chem.Soc.*, 71, 97 (1994) verwiesen.

Esterquats, die quaternierte Fettsäuretriethanolaminestersalze darstellen. haben die allgemeine Formel (I), 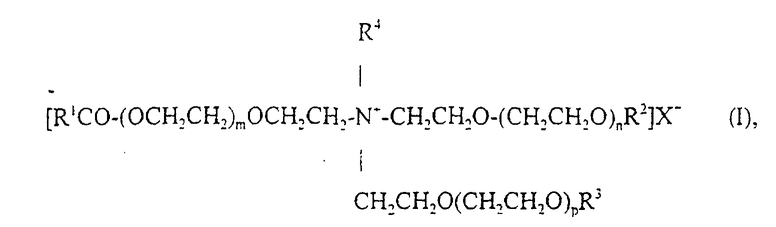 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

Typische Beispiele für diese Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen.

Vorzugsweise werden technische C_{12/14}- und C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt.

Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2, 2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (Iodzahl 0 bis 40) ab.

Aus anwendungstechnischer Sicht haben sich quaternierte Fettsäuretriethanolamin-estersalze der Formel (I) als besonders vorteilhaft erwiesen, in der R¹CO fiir einen Acylrest mit 16 bis 18 Kohlenstoffatomen. R² für R¹CO, R³ für Wasserstoff, R⁴ für eine Methylgruppe, m, n und p für 0 und X für Methylsulfat steht.

Esterquats, die quaternierte Estersalze von Fettsäuren mit Diethanolalkylaminen darstellen, haben die Formel (II). 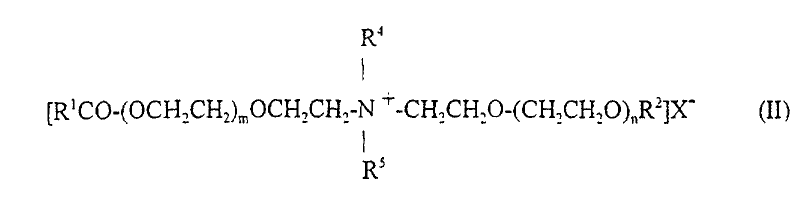 in der R¹CO für einen Acylrest mit 6 bis 22 Kohlenstoffatomen. R² für Wasserstoff oder R¹CO, R⁴ und R5 unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als dritte Gruppe geeigneter Esterquats sind schließlich die quaternierten Estersalze von Fettsäuren mit 1,2-Dihydroxypropyldialkylaminen der Formel (III) zu nennen, 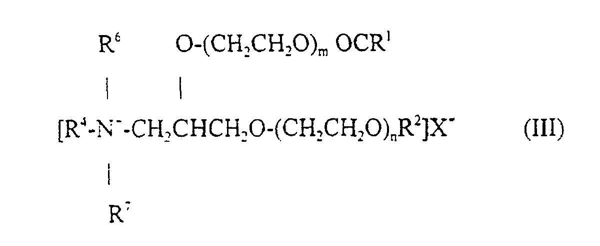 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Ver-esterungsgrades gelten die für (I) genannten Beispiele auch für die Esterquats der Formeln (II) und (III). Esterquats, die in Form 50 bis 90 Gew.- %iger alkoholischer Lösungen im Handel erhältlich sind und problemlos mit Wasser verdünnt werden können, stellen eine bevorzugte eingesetzte Konfektionierungsform dar. In diesem Fall kann Propylenglykol ein bevorzugtes Lösungsmittel für die Esterquats sein.

Die anhand der Formeln I bis III beschriebenen Substanzen stellen naturgemäß Einzelstoffe dar, wobei für den erfindungsgemäßem Einsatz in der Regel auch Gemische dieser Stoffe in Frage kommen können. Ebenso möglich ist gegebenenfalls der Einsatz der technischen Stoffgemische, wie sie aufgrund der Herstellungsverfahren in wechselnder Zusammensetzung erhältlich sind.

Im Rahmen der erfindungsgemäßen Lehre können Esterquats gemäß Formel (II) bevorzugt sein. Eine besonders bevorzugte Verbindung ist das unter der Bezeichnung Armocare® VGH-70 im Handel erhältliche N,N-Bis(2-Palmitoyloxyethyl)dimethylammoniumchlorid.

Eine weitere erfindungsgemäße Gruppe von konditionierenden Wirkstoffen sind die Alkylamidoamine der allgemeinen Formel (IV).

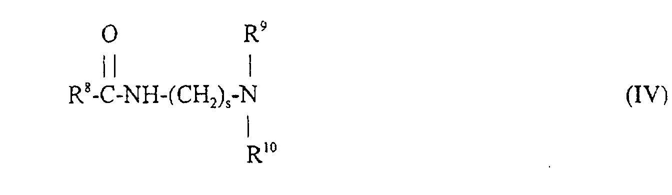

Hierbei steht R⁸-CO für einen aliphatischen, linearen oder verzweigten Acylrest mit 6 bis 22 Kohlenstoffatomen und 0, 1, 2 oder 3 Doppelbindungen, der in der Regel aus einer natürlich vorkommenden oder synthetisch hergestellten Fettsäure stammt. R⁹ und R¹⁰ stellen gleiche oder unterschiedliche Alkylreste dar, die gegebenenfalls funktionelle Gruppen wie Hydroxygruppen, Estergruppen, Amin-, Amid- oder sonstige polare Substituenten tragen können, wobei jedoch in der Regel unsubstituierte Kohlenwasserstoffreste, die allenfalls eine oder mehrere Verzweigungen aufweisen, bevorzugt sind. Insbesondere bevorzugt sind jedoch kurzkettige Kohlenwasserstoffreste mit 1 bis 6 C-Atomen, wobei Ethyl- und Methylreste erfindungsgemäß ganz besonders bevorzugt sind. Die Zahl s ist eine ganze Zahl von 1 bis 10, wobei Werte für n von 2 bis 7, insbesondere von 2 bis 4, bevorzugt sind.

Die Alkylaminoamine werden üblicherweise durch Amidierung natürlicher oder synthetischer Fettsäuren und Fettsäureschnitte mit Dialkylaminoaminen hergestellt. Typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Weiterhin können erfindungsgemäß als konditionierende Wirkstoffe kationische Tenside vom Typ der quartären Ammoniumverbindungen eingesetzt werden. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, Behenyltrimethylammoniummethosulfat sowie die unter den INCI-Bezeichnungen Quaternium-27 und Quaternium-83 bekannten Imidazolium-Verbindungen.

Ebenfalls als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR® 400 sind bevorzugte quatemierte Cellulose-Derivate.
- Polymere Dimethyldiallylammoniumsalze und deren Copolymere mit Estern und Amiden von Acrylsäure und Methacrylsäure. Die unter den Bezeichnungen Merquat® 100 (Poly(dimethyldiallylammoniumchlorid)) und Merquat® 550 (Dimethyldiallylammoniumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat® 734 und Gafquat® 755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquatemium 2,
- Polyquatemium 17,
- Polyquaternium 18 und
- Polyquatemium 27 bekannten Polymeren mit quartären Stickstoffatomen in der Polymerhauptkette.

Besonders bevorzugt sind kationische Polymere der vier erstgenannten Gruppen, insbesondere der Gruppen 3 und 4.

Als konditionierende Wirkstoffe weiterhin geeignet sind Silikonöle, insbesondere Dialkylund Alkylarylsiloxane, wie beispielsweise Dimethylpolysiloxan und Methylphenyl-polysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie die Handelsprodukte DC 344 und DC 345 von Dow Corning, Q2-7224 (Hersteller: Dow Corning; ein stabilisiertes Trimethyl-silylamodimethicon), Dow Corning® 929 Emulsion (enthaltend ein hydroxyl-amino-modifiziertes Silicon, das auch als Amodimethicone bezeichnet wird). SM-2059 (Hersteller: General Electric). SLM-55067 (Hersteller: Wacker) sowie Abil® -Quat 3270 und 3272 (Hersteller: Th. Goldschmidt; diquatemäre Polydimethylsiloxane, Quaternium-80).

Ebenfalls einsetzbar als konditionierende Wirkstoffe sind Paraffinöle sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Ebenfalls neu und daher ein weiterer Gegenstand der Erfindung sind auf der Faser verbleibende Mittel zur Behandlung keratinischer Fasern, dadurch gekennzeichnet, daß sie eine Wirkstoffkombination enthalten, die besteht aus
- mindestens zwei faserstrukturverbessernden Wirkstoffen A1 und A2, wobei A1 ausgewählt ist aus Panthenol und den physiologisch verträglichen Derivaten des Panthenols und A2 ein Pflanzenextrakt oder ein Honigextrakt ist, und
- mindestens einem konditionierenden Wirkstoff B, der ausgewählt ist aus der Gruppe, die von Esterquats und Alkylamidoaminen gebildet wird.

Weiterhin wurde gefunden, daß sich die genannten Eigenschaften des Haares in überraschend starkem Umfang verbessern lassen, wenn das Mittel weiterhin ein kationisches Polymeres enthält. Dies ist unabhängig davon, ob es sich um ein auf dem Haar verbleibendes Mittel handelt oder um ein Mittel, das nach kurzer Einwirkzeit ausgespült wird,

Ein weiterer Gegenstand der Erfindung sind daher Mittel zur Behandlung keratinischer Fasern dadurch gekennzeichnet, daß sie eine Wirkstoffkombination enthalten, die besteht aus
- mindestens zwei faserstrukturverbessernden Wirkstoffen A1 und A2, wobei A1 ausgewählt ist aus Panthenol und den physiologisch verträglichen Derivaten des Panthenols und A2 ein Pflanzenextrakt oder ein Honigextrakt ist, und
- mindestens zwei konditionierenden Wirkstoffen B1 und B2, wobei B1 ausgewählt ist aus der Gruppe, die von Esterquats und Alkylamidoaminen gebildet wird, und B2 ausgewählt ist aus den kationischen Polymeren.

Die folgenden Ausführungen gelten prinzipiell sowohl für die auf der Faser, insbesondere dem Haar, verbleibenden Mittel gemäß Anspruch 7 als auch für die Mittel gemäß Anspruch 8. Abweichungen können aber für Mittel gelten, von denen dem Fachmann bekannt ist, daß sie nach kurzer Einwirkzeit grundsätzlich wieder ausgespült werden, wie z. B. Oxidationsfärbemittel, Blondiermittel und Wellotionen.

Bezüglich der Einzelheiten betreffend die faserstrukturverbessernden bzw, konditionierenden Wirkstoffe sowie die bevorzugten Verbindungen wird ausdrücklich auf die oben gemachten Ausführungen verwiesen.

Die erfindungsgemäßen Mittel können auf wäßriger, wäßrig-alkoholischer oder alkoholischer Basis formuliert sein. Als Alkohole kommen dabei insbesondere niedere Alkohole wie Ethanol und Isopropanol in Betracht. Wäßrig-alkoholische Grundlagen können dabei Wasser und Alkohol, bevorzugt in einem Verhältnis von 1:5 bis 5:1, enthalten.

Panthenol und seine Derivate sind in den erfindungsgemäßen Mitteln bevorzugt in Mengen von 0.05 - 10 Gew.-%, bezogen auf das gesamte Mittel, enthalten. Mengen von 0,1 - 5 Gew.-% sind besonders bevorzugt.

Pflanzenextrakte werden in erfindungsgemäßen Mitteln bevorzugt in Mengen von 0,1 - 20 Gew.-%, insbesondere in Mengen von 0,2 - 8 Gew.-% eingesetzt. Mengen von 0,5 - 5 Gew.-% können ganz besonders bevorzugt sein. Diese Mengenangaben sind zum einen bezogen auf das gesamte erfindungsgemäße Mittel, zum anderen auf den Pflanzenextrakt in der Form, in der dieser dem Mittel zugegeben wird. Dabei kann es sich, wie bereits oben ausgeführt, sowohl um einen reinen Pflanzenextrakt als auch um eine Lösung mit üblicherweise 2-80 Gew.-% Aktivsubstanz handeln.

Bezüglich der Mengenangaben und der Konfektionierungsform der Honigextrakte gilt das für Pflanzenextrakte gesagte, wobei Extrakte mit 1-10 Gew.-%, insbesondere 3-5 Gew.-%, Aktivsubstanz bevorzugt sein können.

Esterquats und Alkylamidoamine können in den erfindungsgemäßen Zubereitungen unabhängig voneinander in Mengen 0.1 - 20 Gew.-%, bezogen auf das gesamte Mittel, enthalten sein. Zur Faserbehandlung im Textilbereich können Mengen von jeweils 0.5 - 15 Gew.-%. insbesondere 5 - 15 Gew.-%, bevorzugt sein. Bei Mitteln zur Behandlung menschlicher Haare werden bevorzugt Wirkstoffkonzentrationen von 0,1 - 7 Gew.-%, insbesondere 0.2 - 5 Gew.-%, eingesetzt. Besonders gute Ergebnisse lassen sich bei Wirkstoffkonzentrationen von 0.2 bis 2 Gew.-% erzielen. Werden die Mittel zur Haarbehandlung allerdings in Form von Konzentraten formuliert, so können die genannten konditionierenden Wirkstoffe in diesen Mittel auch in Mengen von jeweils 2 - 20 Gew.-%, bevorzugt 3 - 14 Gew.-% und insbesondere in 5 - 12 Gew.-%, vorliegen.

Üblicherweise liegen Esterquats und Alkylamidoamine, soweit beide in den erfindungsgemäßen Mitteln enthalten sind, in Mengenverhältnissen von 10:1 bis 1:10, vor, Ein Mischungsverhältnis von 5:1 bis 1:5 oder sogar von 3:1 bis 1:3 kann bevorzugt sein.

Kationische Polymere als konditionierende Wirkstoffe liegen in den erfindungsgemäßen Mitteln bevorzugt in Mengen von 0,02 - 5 Gew.-%, insbesondere 0,04 - 1.0 Gew.-%, bezogen auf das gesamte Mittel, vor.

Der pH-Wert der erfindungsgemäßen Zubereitungen kann prinzipiell zwischen 2 - 11 liegen, wobei der Fachmann ihm bekannte Instabilitäten, beispielsweise des Grundkörpers Panthenol im alkalischen Milieu, berücksichtigen wird. Der pH-Wert der erfindungsgemäßen Mittel liegt bevorzugt zwischen 2 und 7, wobei Werte von 3 bis 5, besonders bevorzugt sind. Zur Einstellung dieses pH-Wertes kann praktisch jede für kosmetische Zwecke verwendbare Säure verwendet werden. Üblicherweise werden Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt.

Neben den erfindungsgemäß zwingend erforderlichen Bestandteilen können die erfindungsgemäßen Mittel prinzipiell alle weiteren, dem Fachmann für solche kosmetischen Mittel bekannte Komponenten enthalten.

Eine wichtige Gruppe dieser fakultativen Komponenten sind, insbesondere für Haarkuren, Fettalkohole mit 8 bis 22 C-Atomen. Die eingesetzten Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Palmitylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estem der Fettsäuren durch Reduktion ausgegangen werden kann. Erfindungsgemäß einsetzbar sind ebenfalls solche Fettalkoholschnitte. die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Die Fettalkohole werden üblicherweise in Mengen von 0,01 bis 15 Gew.-%, bevorzugt von 0,1 bis 10 Gew.-% und besonders bevorzugt von 0,3 bis 6 Gew.-%, bezogen auf die gesamte Zubereitung, eingesetzt.

Neben den beschriebenen Fettalkoholen können auch deren Alkoxylierungsprodukte in Mengen von 0,1 bis 15 Gew.-% zum Einsatz kommen, insbesondere die Ethoxylierungsprodukte der Fettalkohole mit 8 bis 22 C-Atomen. Besonders bevorzugt ist hierbei der Einsatz von ethoxylierten, unverzweigten Fettalkoholen mit 8 bis 20 C-Atomen. wie sie aus der Reduktion und anschließenden Ethoxylierung natürlich vorkommender Fette und Öle, wie beispielsweise Kokosöl, Palmöl, Palmkernöl oder Sonnenblumenöl erhältlich sind.

Die erfindungsgemäßen Zubereitungen können gegebenenfalls noch ein oder mehrere nichtionische Tenside enthalten. Typische Beispiele sind Alkylphenolpolyglycolether, Fettsäurepolyglycolester, Fettsäureamidpolygylcol-ether, Fettaminpolyglycolether, alkoxylierte Triglyceride, wie insbesondere ethoxyliertes Rizinusöl, Alk(en)yloligoglucoside, Fettsäure-N-alkylglucamide, Polyolfettsäureester, Zuckerester, Sorbitanester und Polysorbate. Sofern die nichtionischen Tenside Polyglycoletherketten enthalten, können sie eine konventionelle oder eingeengte Homologenverteilung aufweisen.

Eine weitere Substanzgruppe, die den erfindungsgemäßen Zubereitungen vorteilhafte Eigenschaften in Bezug auf Pflegewirkung und kosmetischen Effekt verleiht, sind die Monoester der Fettsäuren mit Alkoholen mit 6 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Die Fettsäuremonoester können in Mengen von 0.1 bis 5 Gew.-%, bevorzugt 0.8 bis 3 Gew.-%, bezogen auf die gesamte Zubereitung, in den Zubereitungen vorhanden sein.

Die erfindungsgemäße Wirkstoffkombination kann sowohl in Form einer separaten Formulierung oder als zusätzliche Komponenten in anderen Mitteln auf das Haar aufgebracht werden.

Gemäß einer ersten Ausführungsform werden die erfindungsgemäßen Zubereitungen als Haarspülung oder als Haarkur formuliert. Haarspülungen werden in der Regel so formuliert, daß ein Ausspülen der Wirkstoffe nach der gewünschten Einwirkzeit mit Wasser oder einem zumindest überwiegend wasserhaltigen Mittel vorgesehen ist. Die Kontaktzeit mit dem Haar ist in der Regel kurz. Haarkuren enthalten die Wirkstoffkombination in einer höheren Konzentration als Haarspülungen und sind zur Behandlung stark geschädigten Haares vorgesehen. Die Einwirkzeit kann kurz sein. beispielsweise in der Größenordnung der Einwirkzeit von Haarspülungen, sie kann jedoch auch je nach Grad der Schädigung des Haares bis zu 20 Minuten betragen. Auch die erfindungsgemäßen Haarkuren können nach Ablauf dieser Einwirkzeit mit Wasser oder einem zumindest überwiegend wasserhaltigen Mittel ausgespült werden; sie können jedoch auch auf dem Haar belassen werden. Diese Mittel können in einer bevorzugten Variante als Schaumaerosole formuliert werden. Dazu können die Mittel Treibgase enthalten. Gemäß einer ersten bevorzugten Ausführungsform wird das treibgashaltige Mittel in einer Glas- oder PET-Flasche konfektioniert. Mit dieser Ausführungsform lassen sich besonders feine, cremige Schäume, die auch als "micro mousse" bezeichnet werden, erhalten. Eine weitere bevorzugte Ausführungsform ist die Formulierung als Pumpspray mit Luft als Treibmittel.

Gemäß weiteren Ausführungsformen kann es sich bei den erfindungsgemäßen Mitteln beispielsweise um reinigende Mittel wie Shampoos, festigende Mitteln wie Haarfestiger. Haarsprays und Fönwellen, dauerhaften Verformungsmitteln wie Dauerwell- und Fixiermittel, farbverändernden Mittel wie Blondiermitteln, Oxidationsfärbemitteln und Tönungsmitteln auf Basis direktziehender Farbstoffe, Haarwässer und Haarspitzenfluids handeln. Entsprechend können die Zubereitungen als Lösungen, Emulsionen, Gele, Cremes, Aerosole oder Lotionen formuliert werden. Sofern diese Mittel Komponenten enthalten. die zusammen mit einem oder mehreren Bestandteil(en) der erfindungsgemäßen Wirkstoffkombination nicht lagerstabil formuliert werden können, ist es möglich, diese erfindungsgemäße Wirkstoffkomponente oder Wirkstoffkombination dem Mittel in Form einer separaten Formulierung beizugeben und dem Mittel erst unmittelbar vor der Anwendung zuzumischen.

Weitere übliche Bestandteile der erfindungsgemäßen Zubereitungen können somit sein:
- Anionische, zwitterionische, amphotere und nichtionische Polymere wie beispielsweise Vinylacetat/Crotonsäure-Copolymere, Polydimethylsiloxane, Vinylpyrrolidon/Vinylacrylat-Copolymere, Vinylacetat/Butylmaleat/Isobornyl-acrylat-Copolymere, Methylvinylether/Maleinsäureanhydrid-Copolymere und deren Ester, unvernetzte und mit Polyolen vernetzte Polyacrylsäuren, Acryl-amidopropyl-trimethylammomumchlorid/Acrylat-Copolymere, Octylacrylamid/Methyl-methacrylat/tent.Butylaminoethylmethacrylat/2-Hydroxypropylmethacrylat-Copolymere. Polyvinylpyrrolidon, Vinylpyrrolidon/Vinylacetat-Copoly-mere, Vinylpyrrolidon/Dimethylaminoethylmethacrylat/Vinylcaprolactam-Terpolymere sowie gegebenfalls deri-vatisierte Celluloseether.
- anionische Tenside wie insbesondere Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen.
- zwitterionische Tenside, insbesondere die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.
- ampholytische Tenside wie N-Alkylglycine, N-Alkylpropionsäuren, N-Alkylamino buttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether.
- quarternierte Amine wie Methyl-1-alkylamidoethyl-2-alkylimidazolinium-methosulfat
- Entschäumer wie Silikone,
- Verdickungsmittel wie Agar-Agar, Guar-Gum, Alginate, Xanthan-Gum, Gelatine, Pektine, Hydroxyethylcellulose sowie Polyacrylamide und deren Copolymere,
- Strukturanten wie Maleinsäure,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe zum Anfärben des Mittels,
- Antischuppenwirkstoffe wie Piroctone Olamine, Zink Omadine und Climbazol,
- weitere Substanzen zur Einstellung des pH-Wertes
- Wirkstoffe wie Allantoin, Pyrrolidoncarbonsäuren, Vitamine und Bisabolol,
- Lichtschutzmittel,
- Konsistenzgeber wie Zuckerester, Polyolester oder Polyolalkylether,
- Fette und Wachse, wie Walrat, Bienenwachs, Montanwachs und Paraffine,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere.
- Perlglanzmittel wie Ethylenglykolmono- und -distearat sowie PEG-3-distearat,
- Komplexbildner wie EDTA, NTA, β-Alanindiessigsäure und Phosphonsäuren,
- direktziehende Farbstoffe
- sogenannte Kuppler- und Entwicklerkomponenten als Oxidationsfarbstoffvorprodukte,
- Reduktionsmittel wie z. B. Thioglykolsäure und deren Derivate, Thiomilchsäure, Cysteamin, Thioäpfelsäure und α-Mercaptoethansulfonsäure,
- Oxidationsmittel wie Wasserstoffperoxid. Kaliumbromat und Natriumbromat,
- Treibmittel wie Propan-Butan-Gemische, N₂O, Dimethylether, CO₂, N₂ und Luft sowie
- Antioxidantien.

### Formulierungsbeispiele

Alle Mengenangaben sind, soweit nicht anders vermerkt, Gewichtsteile.

| 1. Sprüh-Kur, auf dem Haar verbleibend | |
|---|---|
| Tegoamid® S 18¹ | 0,8 |
| Armocare® VGH-70² | 0,3 |
| D-Panthenol 75 L³ | 0,2 |
| Gafquat® 755⁴ | 0,3 |
| Dehyquart® A⁵ | 0,2 |
| Cremophor® RH 40⁶ | 0,2 |
| Biocorno®⁷ | 0,8 |
| Ethanol (96%) | 10,0 |
| Citronensäure | ad pH 4,0 |
| Wasser | ad 100,0 |

| | |
|---|---|
| ¹ N,N-Dimethyl-N'-stearoyl-1,3-diaminopropan (INCI-Bezeichnung: Stearamidopropyl Dimethylamine) (GOLDSCHMIDT) | |
| ² N,N-Bis(2-Palmitoyloxyethyl)dimethylammoniumchlorid (70 % Aktivsubstanz in Propylenglykol) (AKZO NOBEL) | |
| ³ D-Panthenylalkohol (75 % Aktivsubstanz in Wasser) (HOFFMANN LA-ROCHE) | |
| ⁴ Dimethylaminoethylmethacrylat-Vinylpyrrolidon-Copolymer, mit Diethylsulfat quaterniert (19 % Aktivsubstanz in Wasser; INCI-Bezeichnung: Polyquaternium-11) (GAF) | |
| ⁵ Trimethylhexadecylammoniumchlorid (ca. 25 % Aktivsubstanz in Wasser; INCI-bezeichnung: Cetrimonium Chloride) (HENKEL) | |
| ⁶ hydriertes Rizinusöl + 45 EO (INCI-Bezeichnung: PEG-40 Hydrogenated Castor Oil) (BASF) | |
| ⁷ Mischung aus Maiskeimöl und Getreidekeimöl (INCI-Bezeichnung: Corn Germ Oil) (SYNPHARMA) | |

| 2. Sprüh-Kur. auf dem Haar verbleibend | |
|---|---|
| Tegoamid®D 5040⁸ | 0,5 |
| Armocare®VGH-70 | 0,8 |
| oder Dehyquart® L-80^{2a} | 0,8 |
| D-Panthenol 75 L | 0,5 |
| Luviquat®FC 370⁹ | 0,5 |
| Dehyquart®A | 0,1 |
| Eumulgin®B2¹⁰ | 0,4 |
| Dow Corning®Q2-5220¹¹ | 0,2 |
| Herbasol®Extrakt COS¹² | 0,6 |
| Ethanol (96%) | 50,0 |
| Milchsäure | ad pH 4,5 |
| Wasser | ad 100,0 |

| | |
|---|---|
| ^{2a} Bis(Cocoyl)ethyl-hydroxyethyl-methyl-ammonium-methosulfat (ca. 77 % Aktivsubstanz in Propylenglykol; INCI-Bezeichnung: Dicocoylethyl Hydroxyethylmonium methosulfate, propylene glycol) (HENKEL) | |
| ⁸ Dimethylaminopropylkokosfettsäureamid (INCI-Bezeichnung: Cocamidopropyl Dimethylamine) (GOLDSCHMIDT) | |
| ⁹ Vinylimidazoliummethochlorid-Vinylpyrrolidon-Copolymerisat (30:70) (40 % Aktivsubstanz in Wasser; INCI-Bezeichnung: Polyquaternium-16) (BASF) | |
| ¹⁰ Cetylstearylalkohol + 20 EO (INCI-Bezeichnung: Ceteareth-20) (HENKEL) | |
| ¹¹ Silicon-Glykol-Copolymer (INCI-Bezeichnung: Dimethicone Copolymer) (DOW CORNING) | |
| ¹² Wasser/Propylenglykol-Extrakt aus Akazienhonig (3-5 % Aktivsubstanz in Wasser/PG; INCI-Bezeichnung: Aqua, Propylene Glycol, MEL) (COSMETO-CHEM). | |

| 3. Haar-Kur, abzuspülen | |
|---|---|
| Tegoamid® S 18 | 0,8 |
| Dehyquart® F75¹³ | 1,2 |
| D-Panthenol 75 L | 0.6 |
| Gafquat® 755N¹⁴ | 0,3 |
| Cremophor® RH 40 | 0,3 |
| Lanette® O¹⁵ | 4,3 |
| Isopropylmyristat | 1,0 |
| Incroquat® BTQ-25-C¹⁶ | 0,5 |
| Herbasol® Extrakt Mango¹⁷ | 0,4 |
| Herbasol® Extrakt Aloe entfärbt¹⁸ | 0,4 |
| Konservierung | q.s. |
| Citronensäure | ad pH 4,0 |
| Wasser | ad 100,0 |

| | |
|---|---|
| ¹³ Fettalkohole-Methyltriethanolammoniummethylsulfatdialkylester-Gemisch (INCI-Bezeichnung: Distearoylethyl Hydroxyethylmonium Methosulfate (and) Cetearyl Alcohol) (HENKEL) | |
| ¹⁴ Vinylpyrrolidon-Dimethylaminoethylmethacrylat-Copolymer, mit Diethylsulfat quaterniert (19 % Aktivsubstanz in Wasser; INCI-Bezeichnung: Polyquaternium-11) (GAF) | |
| ¹⁵ C16-18-Fettalkohol (INCI-Bezeichnung: Cetearyl Alcohol) (HENKEL) | |
| ¹⁶ N,N,N-Trimethyl-N-behenylammonium-methosulfat (25 % Aktivsubstanz in Hexadecanol) (CRODA) | |
| ¹⁷ Wasser/Propylenglykol-Extrakt aus Mango (5-8 % Aktivsubstanz in PG/Wasser; INCI-Bezeichnung: Water, Propylene Glykol, Magnifera Indica) (COSMETO-CHEM) | |
| ¹⁸ Wasser/Propylenglykol-Extrakt aus Aloe Vera (2-4 % Aktivsubstanz; INCI-Bezeichnung: Aqua, Propylene Glycol, Aloe Barbadensis) (COSMETOCHEM) | |

| 4. Haar-Spülung | |
|---|---|
| Tegoamid® S 18 | 0,8 |
| Dehyquart® F75 | 1,0 |
| D-Panthenol 75 L | 0,3 |
| Luviquat® FC 370 | 0,6 |
| Lanette® O | 2,0 |
| Dehyquart® A | 0,4 |
| Eumulgin® B2 | 0,4 |
| Novaplant Ginseng spezial¹⁹ | 0,6 |
| Konservierung | q.s. |
| Citronensäure | ad pH 4,2 |
| Wasser | ad 100,0 |

| | |
|---|---|
| ¹⁹ Ginseng-Extrakt (9,5 - 13 % Aktivsubstanz in Wasser/Butylenglykol; INCI-Bezeichnung: Aqua, Butylene Glycol, Panax Ginseng) (NOVAROM) | |

| 5. Haar-Festiger | |
|---|---|
| Tegoamid® S 18 | 0,2 |
| Armocare® VGH-70 | 0,2 |
| D-Panthenol 75 L | 0,2 |
| Luviquat® FC 550²⁰ | 0,3 |
| Dehyquart® SP²¹ | 0,2 |
| Luviskol® VA 64 W²² | 7,0 |
| Uvinul® MS 40²³ | 0,1 |
| Cremophor® RH 40 | 0,1 |
| Parfümöl | 0,1 |
| Ethanol (96%) | 15,0 |
| Wasser | ad 100,0 |

| | |
|---|---|
| ²⁰ Vinylimidazoliummethochlorid-Vinylpyrrolidon-Copolymerisat (50:50) (ca. 40 % Aktivsubstanz in Wasser; INCI-Bezeichnung: Polyquaternium-16) (BASF) | |
| ²¹ Oxyethylalkylammoniumphosphat (ca. 50 % Aktivsubstanz in Wasser; INCI-Bezeichnung: Quaternium 52) (HENKEL) | |
| ²² Vinylpyrrolidon-Vinylacetat-Copolymer (60:40) (50 % Aktivsubstanz in Wasser; INCI-Bezeichnung: PVP/VA-Copolymer) (BASF) | |
| ²³ 2-Hydroxy-4-methoxybenzophenon-5-sulfonsäure (INCI-Bezeichnung: Benzophenone-4) (BASF) | |

## Patentansprüche

1. Verwendung einer Wirkstoffkombination, bestehend aus
- mindestens einem faserstrukturverbessernden Wirkstoff A und
- mindestens einem konditionierenden Wirkstoff B, ausgewählt aus der Gruppe, die aus Esterquats und Alkylamidoaminen gebildet wird,
zum Schutz von keratinischen Fasern vor Schäden durch Hitzeeinwirkung

2. Verwendung nach Anspruch 1, **dadurch gekennzeichnet, daß** der Wirkstoff A ausgewählt ist der Gruppe, die von Panthenol, physiologisch verträglichen Panthenol-Derivaten, Pflanzenextrakten und Honig gebildet wird.

3. Verwendung nach Anspruch 2, **dadurch gekennzeichnet, daß** der Wirkstoff A Panthenol ist.

4. Verwendung nach Anspruch 2, **dadurch gekennzeichnet, daß** der Wirkstoff A ein wäßrig-alkoholischer Pflanzenextrakt ist.

5. Verwendung nach Anspruch 4, **dadurch gekennzeichnet, daß** der Pflanzenextrakt ausgewählt ist aus Extrakten von Mandel, Aloe Vera, Kokosnuß, Mango, Aprikose, Limone, Weizen, Kiwi und Melone.

6. Verwendung nach einem der Ansprüche 1 bis 5, **dadurch gekennzeichnet, daß** der Wirkstoff B ein Esterquat ist.

7. Auf der Faser verbleibendes Mittel zur Behandlung keratinischer Fasern, **dadurch gekennzeichnet, daß** es eine Wirkstoffkombination enthält, die besteht aus mindestens zwei faserstrukturverbessernden Wirkstoffen A1 und A2, wobei A1 ausgewählt ist aus Panthenol und den physiologisch verträglichen Derivaten des Panthenols und A2 ein Pflanzenextrakt oder ein Honigextrakt ist, und mindestens einem konditionierenden Wirkstoff B, der ausgewählt ist aus der Gruppe, die von Esterquats und Alkylamidoaminen gebildet wird.

8. Mittel zur Behandlung keratinischer Fasern, **dadurch gekennzeichnet, daß** es eine Wirkstoffkombination enthält, die besteht aus
- mindestens zwei faserstrukturverbessernden Wirkstoffen A1 und A2, wobei A1 ausgewählt ist aus Panthenol und den physiologisch verträglichen Derivaten des Panthenols und A2 ein Pflanzenextrakt oder ein Honigextrakt ist, und
- mindestens zwei konditionierenden Wirkstoffen B 1 and B2, wobei B1 ausgewählt ist aus der Gruppe, die von Esterquats und Alkylamidoaminen gebildet wird, und B2 ausgewählt ist aus den kationischen Polymeren.

9. Mittel nach Anspruch 7 oder 8, **dadurch gekennzeichnet, daß** es auf wäßrig-alkoholischer Basis formuliert ist, wobei das Verhältnis Wasser : niederer Alkohol 5:1 bis 1:5 beträgt.

10. Mittel nach einem der Ansprüche 7 bis 9, **dadurch gekennzeichnet, daß** es einen pH-Wert von 2 bis 7 aufweist.

11. Mittel nach einem der Ansprüche 8 bis 10, **dadurch gekennzeichnet, daß** es als Pump-Spray mit Luft als Treibmittel formuliert ist.

## Claims

1. The use of a combination of active agents consisting of
- at least one fibre-structure-improving agent A and
- at least one conditioning agent B selected from the group consisting of esterquats and alkyl amidoamines
for protecting keratin fibres against damage by heat.

2. The use claimed in claim 1, **characterized in that** the agent A is selected from the group consisting of panthenol, physiologically compatible panthenol derivatives, plant extracts and honey.

3. The use claimed in claim 2, **characterized in that** the agent A is panthenol.

4. The use claimed in claim 2, **characterized in that** the agent A is a water/alcohol plant extract.

5. The use claimed in claim 4, **characterized in that** the plant extract is selected from extracts of almond, aloe vera, coconut, mango, apricot, lemon, wheat, kiwi and melon.

6. The use claimed in any of claims 1 to 5, **characterized in that** the agent B is an esterquat.

7. A leave-on preparation for treating keratin fibres, **characterized in that** it contains a combination of active agents consisting of at least two fibre-structure-improving agents A1 and A2, A1 being selected from panthenol and physiologically compatible derivatives of panthenol and A2 being a plant extract or a honey extract, and at least one conditioning agent B selected from the group consisting of esterquats and alkyl amidoamines.

8. A preparation for treating keratin fibres, **characterized in that** it contains a combination of active agents consisting of
- at least two fibre-structure-improving agents A1 and A2, A1 being selected from panthenol and physiologically compatible derivatives of panthenol and A2 being a plant extract or a honey extract, and
- at least two conditioning agents B1 and B2, B1 being selected from the group consisting of esterquats and alkyl amidoamines and B2 being selected from the cationic polymers.

9. A preparation as claimed in claim 7 or 8, **characterized in that** it is formulated on a water/alcohol basis, the ratio of water to lower alcohol being 5:1 to 1:5.

10. A preparation as claimed in any of claims 7 to 9, **characterized in that** it has a pH value of 2 to 7.

11. A preparation as claimed in any of claims 8 to 10, **characterized in that** it is formulated as a pump spray with air as the propellant.

## Revendications

1. Utilisation d'une combinaison de substance active constituée de
- au moins une substance active A améliorant la substance des fibres et
- au moins une substance active B conditionnante, choisie dans le groupe constitué par les esterquats et les alkylamidoamines,
pour la protection des fibres kératiniques contre les atteintes dues à l'action de la chaleur.

2. Utilisation selon la revendication 1,
**caractérisée en ce que**
la substance active A est choisie dans le groupe formé par le panthénol, les dérivés de panthénol physiologiquement acceptables, les extraits de plantes et le miel.

3. Utilisation selon la revendication 2,
**caractérisée en ce que**
la substance active A est le panthénol.

4. Utilisation selon la revendication 2,
**caractérisée en ce que**
la substance active A est un extrait de plante alcoolique aqueux.

5. Utilisation selon la revendication 4,
**caractérisé en ce que**
l'extrait de plante est choisi parmi les extraits d'amande, d'aloès, de noix de coco, de mangue, d'abricot, de citron, de blé, de kiwi, de melon.

6. Utilisation selon l'une des revendications 1 à 5,
**caractérisée en ce que**
la substance active B est un esterquat.

7. Agent restant sur les fibres pour le traitement des fibres kératiniques,
**caractérisé en ce qu'**
il contient une combinaison de substances actives qui se compose d'au moins deux substances actives A1 et A2 améliorant la structure des fibres, A1 étant choisi parmi le panthénol et les dérivés physiologiquement acceptables du panthénol et A2 étant un extrait de plante ou un extrait de miel, et d'au moins une substance active conditionnante B, qui est choisie dans le groupe formé par les esterquats et les alkylamidoamines.

8. Agent de traitement des fibres kératiniques,
**caractérisé en ce qu'**
il contient une combinaison de substances actives constituée par
- au moins deux substances actives A1 et A2 améliorant la substance des fibres, A1 étant choisi parmi le panthénol et les dérivés physiologiquement acceptables du panthénol et A2 étant un extrait de plante ou un extrait de miel et
- au moins deux substances actives conditionnantes B1 et B2 et B1 étant choisi dans le groupe formé par les esterquats et les alkylamidoamines, et B2 étant choisi parmi les polymères cationiques.

9. Agent selon les revendications 7 ou 8,
**caractérisé en ce qu'**
il est formulé sur une base alcoolique aqueuse, le rapport eau:alcool inférieur étant de 5:1 à 1:5.

10. Agent selon les revendications 7 à 9,
**caractérisé en ce qu'**
il présente un pH de 2 à 7.

11. Agent selon les revendications 8 à 10,
**caractérisé en ce qu'**
il est formulé en produit de pulvérisation à la pompe avec de l'air comme vecteur.
